# EUROPÄISCHE PATENTSCHRIFT

(11) **EP 1 071 691 B1**
(45) Veröffentlichungstag und Bekanntmachung des Hinweises auf die Patenterteilung: **28.09.2005**
(21) Anmeldenummer: 99907401.6
(22) Anmeldetag: 22.01.1999
(51) Int. Cl.: C07H 1/08

(54) **VERFAHREN ZUR ISOLIERUNG UND AUFREINIGUNG VON NUCLEINSÄUREN**
METHOD FOR ISOLATING AND PURIFYING NUCLEIC ACIDS
PROCEDE POUR ISOLER ET PURIFIER DES ACIDES NUCLEIQUES

(30) Priorität: 04.02.1998 DE 19804243
(43) Veröffentlichungstag der Anmeldung: 31.01.2001
(73) Patentinhaber: Merck Patent GmbH, 64293 Darmstadt (DE)
(72) Erfinder: MICHELSEN, Uwe, D-69469 Weinheim (DE); HOLSCHUH, Karl, D-64342 Seeheim-Jugenheim (DE); HENDRIKS, Robertus, D-69121 Heidelberg (DE)
(86) Internationale Anmeldenummer: PCT/EP1999/000413
(87) Internationale Veröffentlichungsnummer: WO 1999/040098

(56) Entgegenhaltungen:
- EP-A- 0 818 461
- EP-A- 0 897 978
- WO-A-96/41811
- DE-A- 4 321 904

## Beschreibung

Die Erfindung betrifft ein Verfahren zur Isolierung und Aufreinigung von Nucleinsäuren aus Flüssigkeiten mit Hilfe eines festen Trägermaterials.

Es existiert ein umfangreicher Stand der Technik, der die Isolierung von Nucleinsäuren aus Körperflüssigkeiten beschreibt. Ältere Methoden umfassen mehrere Arbeitsschritte: Anreicherung der Nucleinsäure enthaltenden Zellen oder Kompartimente, Lysis derselben, Abtrennung der Protein- und Membranfraktionen, Ausfällen der gereinigten Nucleinsäuren zur Entfernung kontaminierender Chemikalien. Neuere Methoden benutzen Festphasenextraktionen, wobei unter spezifischen Reaktionsbedingungen die Nucleinsäure enthaltenden Kompartimente lysiert und DNA und RNA oder deren Subpopulationen an die Festphase gebunden werden. Spezifische Reaktionsbedingungen sind hochmolare Konzentrationen von bestimmten chaotropen Substanzen oder von wasserlöslichen organischen Lösungsmitteln. Ebenso kann hochmolekulare, genomische DNA eukaryontischer Zellen durch einfache Lysis der Zellen mittels Detergenz und physikalische Interaktion der langen DNA-Fäden mit Mikropartikein oder großporigen Filtern eingefangen und gereinigt werden. Es ist auch bekannt, daß gereinigte Nucleinsäuren in wäßrigem Milieu an Chromatographiematerial wie Hydroxylapatit binden und mit relativ hochmolarem Phosphatpuffer abgelöst werden können.

Aus US 5,234,809 ist ein Verfahren zur Isolierung von Nucleinsäuren bekannt, wobei die Bindung an eine feste Phase in Gegenwart hoher Konzentrationen von chaotropen Substanzen wie Guanidiniumsalzen, Natriumjodid, Natriumthiocyanat oder Harnstoff erfolgt. Die gebundenen Nucleinsäuren werden mit einem eine chaotrope Substanz enthaltenden Waschpuffer gewaschen. Anschließend wird zur Entfernung der hochkonzentrierten Salze mit einer Alkohol und/oder Aceton enthaltenden Waschlösung gewaschen. Die Elution der Nucleinsäuren erfolgt nach sorgfältiger Entfernung der organischen Lösungsmittel mit Wasser oder mit Puffer niedriger lonenstärke. Ähnliche Verfahren sind aus EP 0 572 907 bekannt.

Auch EP 0 818 461 und DE 43 21 904 offenbaren Verfahren zur Isolierung von Nukleinsäuren, bei denen die Nukleinsäuren unter chaotropen Bedingungen an hydroxylgruppenhaltige Trägermaterialien gebunden werden.

Nach WO 96/18731 soll die Bindung an die feste Phase in Gegenwart von Detergenzien unter neutralen Pufferbedingungen erfolgen. Das Verfahren hat jedoch den Nachteil, daß nur langkettige DNA-Moleküle wie genomische DNA eukaryontischer Zellen an die feste Phase gebunden werden können. Kurze DNA-Moleküle wie auch RNA-Moleküle können unter den beschriebenen Pufferbedingungen nicht direkt isoliert werden.

Die beschriebenen Methoden zur Isolierung und Aufreinigung von Nucleinsäuren weisen eine Reihe von Nachteilen auf. Chaotrope Substanzen in hohen Konzentrationen, organische Lösungsmittel und Detergenzien wirken inhibierend auf nachfolgende molekularbiologische Reaktionen, zu deren Zweck die Nucleinsäuren isoliert werden. Intensive Waschschritte oder Trockenschritte sind erforderlich, um diese Inhibitoren quantitativ zu entfernen. Auch für Automationsvorhaben mit dem Ziel, aus einer hohen Anzahl von Proben schnell und reproduzierbar Genmaterial zu isolieren, sind die beschriebenen Chemikalien und Waschschritte hinderlich.

In WO 97/34 909 wird ein Verfahren zur Isolierung von Nucleinsäuren offenbart, bei dem die Nucleinsäuren aus der Probe an einem organischen vernetzten Polymer, das basische Gruppen aufweist, gebunden werden. Für diesen Bindungsschritt sind Zusätze von Detergenzien, chaotropen Substanzen oder organischen Lösungsmitteln nicht notwendig. Jedoch erfolgt die Bindung langsam und erfordert Inkubationszeiten von einer Stunde oder mehr.

Es stellt sich also die Aufgabe, ein Verfahren zur Isolierung und Aufreinigung von Nucleinsäuren bereitzustellen, das ohne störenden Zusatz der genannten Substanzen (z.B. chaotrope Substanzen) ausführbar ist, und das eine kurze Inkubationszeit erfordert. Die isolierten beziehungsweise aufgereinigten Nucleinsäuren (DNA und RNA) sollten sofort nach Elution von der Festphase für molekularbiologische Reaktionen eingesetzt werden können.

Es wurde gefunden, daß bei Verwendung von anorganischen hydroxylgruppenhaltigen oxidischen Materialien eine Bindung von Nucleinsäuren an diese Trägermaterialien auch ohne Zusatz von chaotropen Substanzen möglich ist, wenn durch den Bindungspuffer der pH auf 1 bis 6 erniedrigt wird. Diese Bindung erfolgt schnell, d.h. innerhalb von weniger als 15 Minuten. Nach einem optionalen Waschschritt kann die Nucleinsäure durch einen Elutionspuffer mit einem pH-Wert zwischen 7,5 und 11 in Lösung gebracht werden und steht für weitere molekularbiologische Reaktionen bereit.

Gegenstand der Erfindung ist ein Verfahren zur Isolierung und Aufreinigung von Nucleinsäuren aus flüssigen Proben, gekennzeichnet durch folgende Verfahrensschritte:
a) Bereitstellen einer flüssigen Probe, die Nucleinsäuren enthält;
b) Bereitstellen eines Trägermaterials aus einem anorganischen hydroxylgruppenhaltigen oxidischen Material, das zumindest kristalline oder amorphe Modifikationen von SiO₂ und Silikaten, Zeolithe oder Hydroxylapatite enthält;
c) Verdünnen der Probe aus Schritt a) mit einem sauren Bindungspuffer, der keine Ionen, ionische Detergenzien oder chaotrope Substanzen in Konzentrationen größer 200 mM enthält, so dass der pH-Wert der Probe auf unter pH 6 abgesenkt wird;
d) Behandeln der mittels eines Bindungspuffers angesäuerten Probe aus Schritt c) mit dem Trägermaterial aus Schritt b), wobei die Nucleinsäuren gebunden werden;
e) Abtrennen der Probe und des Bindungspuffers nach Bindung der Nucleinsäuren;
f) Eluieren der in Schritt d) gebundenen Nucleinsäuren mittels einer alkalischen Lösung.

In bevorzugten Ausführungsformen wird ein Waschschritt e1) nach Schritt e) eingefügt, wobei der pH des dabei verwendeten Waschpuffers ≤ 6,5 beträgt. In weiteren bevorzugten Ausführungsformen beträgt der pH des Bindungspuffers zwischen 3 und 6 und des Elutionspuffers 7,5 bis 9. Bevorzugte Trägermaterialien sind Kieselgel und Hydroxylapatit.

Gegenstand der Erfindung sind ferner Reagenzzusammenstellungen für das erfindungsgemäße Verfahren zur Isolierung und Aufreinigung von Nucleinsäuren aus flüssigen Proben. In bevorzugten Ausführungsformen enthalten diese Reagenzzusammenstellungen alle für die Durchführung des Verfahrens notwendigen Bestandteile: Trägermaterial, Bindungspuffer, einen oder mehrere Waschpuffer und Elutionspuffer. Es ist jedoch auch möglich, einzelne dieser Komponenten getrennt zu liefern, oder dem Benutzer die Beschaffung dieser Komponente zu überlassen, so daß auch Reagenzzusammenstellungen beispielsweise ohne den Waschpuffer Gegenstand der Erfindung sind. Somit kann eine erfindungsgemäße Reagenzzusammenstellung auch zwei oder drei Bestandteile ausgewählt aus Trägermaterial, Bindungspuffer, Waschpuffer und Elutionspuffer umfassen, insbesondere beispielsweise Trägermaterial und Bindungspuffer umfassen. Reagenzzusammenstellungen für das erfindungsgemäße Verfahren können ferner zusätzlich Hilfsmittel wie Zentrifugenröhrchen oder Dosierhilfen enthalten.

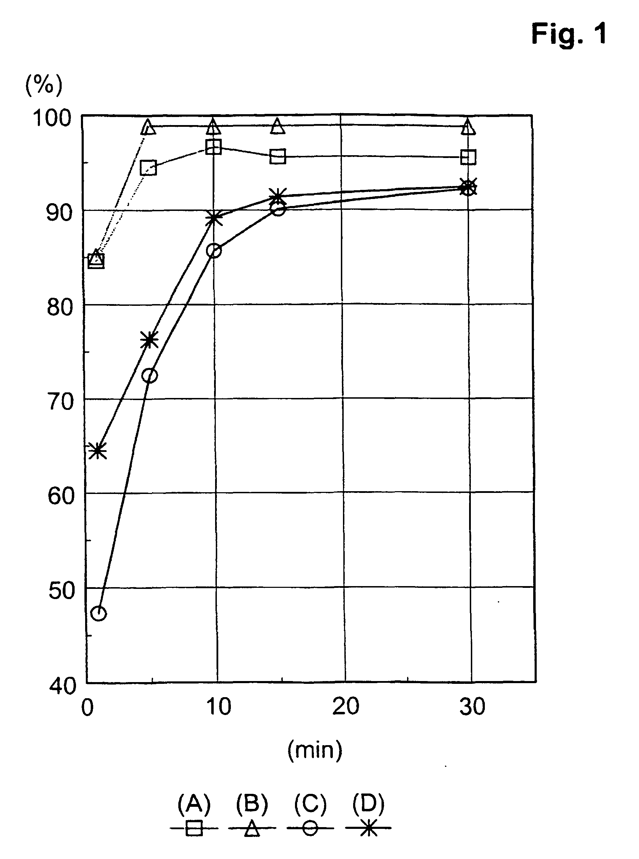

In Abbildung 1 werden die Bindungskinetiken für das erfindungsgemäße Verfahren (Kurven (A) und (B)) mit einem Verfahren entsprechend dem Stand der Technik nach WO 97/34 909 (Kurven (C) und (D)) verglichen. Für die in den Kurven (B) und (D) dargestellten Meßergebnisse wurde der Probe Rinderserumalbumin zugesetzt, um die Verwendung der Verfahren für proteinhaltige Proben zu simulieren. Weitere experimentelle Einzelheiten finden sich in in der Beschreibung zu Vergleichsbeispiel A.

Als Trägermaterialien geeignete anorganischen hydroxylgruppenhaltigen oxidischen Materialien sind aus dem Stand der Technik, beispielsweise aus US 5,234,809 bekannt: Dazu gehören insbesondere kristalline oder amorphe Modifikationen von SiO₂ und Silikaten, d.h. beispielsweise Kieselgel, Kieselgur, Silikatgläser oder Zeolite, weiterhin auch Hydroxylapatite. Bevorzugt als Trägermaterial sind kristalline oder amorphe Modifikationen von SiO₂ und Silikaten.

Die Trägermaterialien können in Form von Beads, Partikeln, Blättern, Gelen, Filtern, Membranen, Fasern, in Kapillaren, Streifen, Röhrchen, Mikrotiterplatten usw. vorliegen. Es können auch entsprechende magnetische Partikel verwendet werden. Die Trägermaterialien können beispielsweise auch als Beschichtung auf Gefäßen aufgebracht sein. Als Äquilibrierungspuffer für das Trägermaterial wird vorzugsweise einer der im folgenden genannten Bindungspuffer verwendet.

Die Bindung der Nucleinsäuren aus der Probe erfolgt durch einfaches Absenken des pH-Wertes auf unter pH 6. Dazu wird ein Bindungspuffer verwendet, der einem pH-Bereich von 1 bis 6, vorzugsweise von 3 bis 5, aufrechterhalten kann. Im bevorzugten pH-Bereich weisen bekanntermaßen die Purinbasen der Nukleinsäuren verbesserte Stabilität auf. Geeignete Puffer sind z.B. Formiat-, Acetat-, Citratpuffer oder andere Puffersysteme, die in dem genannten pH-Bereich ausreichende Pufferkapazität besitzen.

Die Pufferkonzentration sollte im Bereich von 10 bis 200 mM liegen, je nach der Pufferkapazität der zu untersuchenden Probeflüssigkeit. Vorzugsweise wird ein Bindungspuffer der Konzentration von 50 mM verwendet, der einen pH-Wert von ca. 4,5 hat, z.B. ein Puffer aus Essigsäure, der mit Natronlauge, Kalilauge oder mit Tris-Base auf einen pH-Wert zwischen 4 und 5 eingestellt wurde. Dem Bindungspuffer können Nucleaseinhibitoren zugesetzt werden; geeignete Nucleaseinhibitoren sind dem Fachmann bekannt.

Ein Bindungspuffer entsprechend der vorliegenden Erfindung enthält weder Phosphationen, noch andere Ionen in hohen Konzentrationen (> 200 mM), noch ionische Detergenzien oder chaotrope Substanzen.

Bei den Nucleinsäuren enthaltenden Proben handelt es sich um wäßrige Flüssigkeiten, wie Pufferlösungen und Homogenate oder komplexe biologische Flüssigkeiten, wie Blut, Serum, Plasma, Urin usw. oder um Gewebe- und Zelllysate.

Das Trägermaterial mit den daran gebundenen Nucleinsäuren kann mit dem beschriebenen Bindungspuffer oder mit anderen phosphatfreien Puffern gewaschen werden, wobei der pH-Wert dieser Puffer zwischen 4 und 7, vorzugsweise zwischen 4,5 und 6,5 liegen sollte. Die Pufferkonzentration kann geringer sein als beim Bindungspuffer; sie sollte im Bereich von 5 bis 50 mM, vorzugsweise bei etwa 8 bis 15 mM liegen. Gegebenenfalls kann einer der Waschpuffer Zusatzstoffe, beispielsweise Chelatbildner wie EDTA, chaotrope Substanzen und/oder nichtionische Detergenzien enthalten. Die erfindungsgemäß offenbarten Waschpuffer eluieren die gebundenen Nucleinsäuren nicht, selbst wenn diese unter Zusatz von chaotropen Substanzen an das Trägermaterial adsorbiert wurden. In einem Waschpuffer entsprechend der vorliegenden Erfindung sind Zusätze von organischen Lösungsmitteln und/oder chaotropen Substanzen nicht notwendig, um die vorzeitige Elution von Nucleinsäuren zu vermeiden. Jedoch sind Zusätze der genannten Hilfsstoffe in dem Verfahren nach US 5,234,809 notwendig, um die vorzeitige Elution von Nucleinsäuren zu vermeiden. Im Zusammenhang mit der vorliegenden Erfindung können derartige Zusätze verwendet werden, um die isolierten Nucleinsäurepräparationen zu reinigen, beispielsweise um adsorbierte Proteine von dem Träger auszuwaschen. Für derartige Zwecke geeignete Zusätze und deren Konzentrationen sind dem Fachmann geläufig.

Überraschenderweise wurde gefunden, daß die so gereinigte Nucleinsäure durch eine einfache Erhöhung des pH-Wertes auf über 7,5 von der Festphase gelöst werden kann. Der dazu verwendete Elutionspuffer sollte einen pH-Bereich von 7,5 bis 9, vorzugsweise 8 bis 8,5 aufrechterhalten. Geeignete Puffer sind z.B. Tris-HCl-Puffer, Tricin, Bicin und andere Puffer, die in diesem pH-Bereich puffern, vorzugsweise Tris/HCl. Die Pufferkonzentration sollte 5 bis 10 mM, vorzugsweise etwa 8 bis 10 mM betragen. Gegebenenfalls kann der Elutionspuffer Chelatbildner wie EDTA und/oder andere Inhibitoren von Nucleasen enthalten. Die eluierte Nucleinsäure ist direkt, ohne weitere Reinigungsschritte, für molekularbiologische Anwendungen, wie z.B. für Ampifikationsreaktionen einsetzbar.

Das Verfahren zur Isolierung und Aufreinigung von Nucleinsäuren wird entsprechend der vorliegenden Erfindung so durchgeführt, daß z.B. ein die Nucleinsäuren enthaltendes Serum mit dem Bindungspuffer versetzt und in ein Mikrozentrifugenröhrchen gegeben wird, das bereits die äquilibrierte Festphase enthält. Nach einer Inkubationszeit wird zentrifugiert und der Überstand wird verworfen. Mit einem Waschpuffer wird resuspendiert, erneut zentrifugiert und der Überstand erneut verworfen. Es können auch mehrere Waschschritte gegebenenfalls mit Waschpuffern unterschiedlicher Zusammensetzung nacheinander ausgeführt werden. Dementsprechend kann eine erfindungsgemäße Reagenzzusammenstellung auch mehrere Waschpuffer enthalten. Schließlich wird der Elutionspuffer hinzugefügt, die Suspension zentrifugiert und der die Nucleinsäuren enthaltende Überstand in ein neues leeres Röhrchen überführt. Diese Lösung kann dann direkt für weitere Analysenmethoden (PCR, NASBA) eingesetzt werden. Bei der Verwendung von z.B. entsprechenden magnetischen Beads kann die Zentrifugaton durch das Anlegen eines magnetischen Felds ersetzt werden.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die vollständige Offenbarung aller vor- und nachstehend aufgeführten Anmeldungen, Patente und Veröffentlichungen, sowie der korrespondierenden Anmeldung DE 198 04 243.4, eingereicht am 04.02.1998, sind durch Bezugnahme in diese Anmeldung eingeführt.

### Beispiel 1

### Materialien

### Mikrozentrifugenröhrchen

| | |
|---|---|
| Bindungspuffer (BP) | 50 mM Essigsäure/KOH, pH 4,5 |
| Waschpuffer (WP) | 10 mM Tris-HCl, pH 6,5 |
| Elutionspuffer (EP) | 10 mM Tris-HCl, 1 mM EDTA, pH 9,0 |
| DNA oder RNA | 1 ng - 1 µg |
| Partikel | 0,5 g in 1 ml 10 mM Tris-HCl, pH 6,5 |

### Verfahrensschritte

1. 80 µl BP, 1 ng - 1 µg DNA/RNA, 1 µl Partikel (500 mg/ml) werden mit Wasser auf 100 µl aufgefüllt,
2. die Mischung wird 5 Minuten bei Raumtemperatur inkubiert,
3. es wird 10 Sekunden bei 5000 rpm zentrifugiert, der Überstand wird verworfen,
4. die Partikel werden mit 200-500 µl WP resuspendiert,
5. die Schritte 3 und 4 werden wiederholt,
6. Schritt 3 wird wiederholt,
7. es werden 30 µl EP hinzugegeben,
8. es wird 10 Sekunden bei 5000 Rpm zentrifugiert, der Überstand wird in ein neues Röhrchen überführt.

Es werden verschiedene Partikel eingesetzt: Hydroxylapatit (Merck Art. #1.05119) und Silica-Partikel (Merck Art. 1.01193). Diese werden mit und ohne Zusatz von Serum zum Bindungspuffer getestet. Die Konzentration an radioaktiv markierter Lambda-DNA pro Test entspricht etwa 1 ng. Die Radioaktivität ist in cpm 1000 angegeben.

### Pippettierschema [µl]

| **Probe Nr.** | **1** | **2** | **3** | **4** | **K1** | **K2** |
|---|---|---|---|---|---|---|
| **Partikel:** | **H** | **S** | **H** | **S** | | |
| **H: Hydroxylapatit** | | | | | | |
| **S: Silicagel** | | | | | | |
| Bindungspuffer (µl) | 80 | 80 | 80 | 80 | 80 | 80 |
| Serum (µl) | | | 10 | 10 | | |
| Wasser (µl) | 10 | 10 | | | 11 | 11 |
| P-32 λ-DNA (µl) | 9 | 9 | 9 | 9 | 9 | 9 |
| Partikel (µl) | 1 | 1 | 1 | 1 | | |

### Ergebnisse

| **Probe Nr.** | **1** | **2** | **3** | **4** | **K1** | **K2** |
|---|---|---|---|---|---|---|
| Überstand [cpm] | 22 | 14 | 7 | 17 | 179 | 173 |
| gebunden [cpm] | 154 | 162 | 169 | 159 | | |
| 1. Elution [cpm] | 114 | 132 | 94 | 139 | | |
| 5 Min., Tris-Puffer pH 9 | | | | | | |
| 2. Elution [cpm] | 36 | 4 | 56 | 1 | | |
| Phosphat-Puffer pH 7 | | | | | | |
| Rest auf Partikel [cpm] | 5 | 16 | 7 | 4 | | |
| Bindungskapazität [%] | 88 | 92 | 96 | 90 | | |
| Elutionseffizienz [%] | | | | | | |
| 1. Elution | 74 | 81 | 55 | 87 | | |
| 2. Elution | 97 | 83 | 88 | 88 | | |

Die hier benutzten Partikel mit unterschiedlichen chemischen Gruppen an der Oberfläche binden unter den gewählten Bedingungen 80 bis 96 % der markierten DNA. In der Regel wird über 80 % der gebundenen DNA durch einfach pH-Änderung (z.B. Tris-Puffer) wieder eluiert. Ein hochmolarer Phosphat-Puffer (0,5 M) verbessert die Elutionseffizienz bei den Hydroxylapatit-Partikeln.

Bei Verwendung entsprechender magnetischer Partikel wird die Zentrifugation durch das Anlegen eines magnetischen Feldes ersetzt.

### Beispiel 2

Entsprechend Beispiel 1 wird eine Nucleinsäure enthaltende Probe (1 µg) mit steigenden Mengen Serum versetzt. Zur Erhöhung der Pufferkapazität wird der Bindungspuffer bei hohen Serumkonzentrationen 4fach konzentriert eingesetzt (= 200 mM). Die Ausbeute wird über Ethidiumbromid gefärbte Nucleinsäurebanden in Agarosegelen bestimmt. Als Trägermaterial werden Silicapartikel verwendet.

| | | | | | |
|---|---|---|---|---|---|
| Serum [µl] | 0 | 10 | 20 | 30 | 50 |
| 1 x BP [µl] | 80 | 80 | 70 | - | - |
| 4 x BP [µl] | - | - | - | 50 | 40 |
| Ausbeute DNA bzw. RNA | +++ | ++++ | ++++ | ++++ | +++ |

Die Ergebnisse zeigen, daß die Bindung der Nucleinsäuren an Silicapartikel unabhängig von der Serumkonzentration ist.

Der obige Versuch wird unter Verwendung von Hydroxylapatitpartikeln anstelle der Silicapartikel wiederholt; auch bei Verwendung von Hydroxylapatit werden ähnliche Ergebnisse erhalten.

### Beispiel 3

Entsprechend Beispiel 1 werden verschiedene Puffer mit verschiedenen pH-Werten eingesetzt und ihre Eignung als Bindungspuffer getestet. Als Trägermaterial werden Silicapartikel verwendet. DNA sowie RNA (ribosomale Hefe-RNA) wird in einer Endkonzentration von 0.5 µg eingesetzt. Die Ausbeute des Eluats wird über Ethidiumbromid gefärbte Nucleinsäurebanden in Agarosegelen bestimmt.

### Pipettierschema [µl]

| **Probe Nr. Bindungspuffer** | **1** | **2** | **3** | **4** | **5** | **6** | **7** | **8** | **9** | **10** | **11** | **12** | **13** | **14** |
|---|---|---|---|---|---|---|---|---|---|---|---|---|---|---|
| Acetat/KOH pH 4,5 | 80 | 80 | | | | | | | | | | | | |
| Acetat/KOH pH 5.0 | | | 80 | 80 | | | | | | | | | | |
| Acetat/NaOH pH 4,5 | | | | | 80 | 80 | | | | | | | | |
| MES pH 5,5 | | | | | | | 80 | 80 | | | | | | |
| MES pH 6,0 | | | | | | | | | 80 | 80 | | | | |
| MES pH 6,5 | | | | | | | | | | | 80 | 80 | | |
| Tris-HCl pH 7,0 | | | | | | | | | | | | | 80 | 80 |
| Wasser | 15 | 15 | 15 | 15 | 15 | 15 | 15 | 15 | 15 | 15 | 15 | 15 | 15 | 15 |
| DNA/RNA | 4 | 4 | 4 | 4 | 4 | 4 | 4 | 4 | 4 | 4 | 4 | 4 | 4 | 4 |
| Partikel (500 mg/ml) | 1 | 1 | 1 | 1 | 1 | 1 | 1 | 1 | 1 | 1 | 1 | 1 | 1 | 1 |

### Ergebnisse

| | | | | | | | | | | | | | | |
|---|---|---|---|---|---|---|---|---|---|---|---|---|---|---|
| **Ausbeute in %** | **90** | **90** | **80** | **80** | **90** | **90** | **70** | **70** | **50** | **50** | **20** | **20** | **2** | **2** |

Die erhaltenen Ausbeuten wurden unabhängig von dem Trägermaterial erzielt. Die höchsten Ausbeuten wurden bei pH 4,5 erreicht.

Der Versuch wird unter Verwendung von Hydroxylapatitpartikeln anstelle der Silicapartikel wiederholt; es werden ähnliche Ergebnisse wie oben zusammengestellt erhalten.

### Vergleichsbeispiel A

In einem Vergleichsversuch wurden die Bindungskinetiken des erfindungsgemäßen Verfahrens mit dem Verfahren unter Verwendung eines basischen organischen Polymers verglichen. Dazu wurden 1 µg λ Hind III-DNA (³²P-markiert; ca. 100 000 cpm) in 50 mM Kaliumacetat, pH 4,5, mit und ohne Zusatz von 10 mg/l Rinderserumalbumin (BSA), erfindungsgemäß an Silicapartikel, und zum Vergleich an ESTAPOR® -NH₂ Partikel (aminoderivatisiertes, vernetztes organisches Polymer) gebunden. Die Reaktion wird durch die Zugabe der Partikel gestartet; der Meßwert für 0 Minuten wird vor Zugabe der Partikel bestimmt. Durch Messung der Radioaktivität im Überstand wird die Bindungskinetik bestimmt; angegeben ist die Radioaktivität im Überstand:

| **Zeit** | **erfindungsgemäß organisches Polymer** | | | |
|---|---|---|---|---|
| | **(cpm / 1000)** | | | |
| **(min)** | **ohne BSA** | **mit BSA** | **ohne BSA** | **mit BSA** |
| **0** | 91 | 94 | 91 | 93 |
| **1** | 14 | 14 | 48 | 33 |
| **5** | 5 | 1 | 25 | 22 |
| **10** | 3 | 1 | 13 | 10 |
| **15** | 4 | 1 | 9 | 8 |
| **30** | 4 | 1 | 7 | 7 |
| **60** | 4 | 1 | 4 | 3 |
| **120** | 4 | 1 | 3 | 2 |

Es zeigt sich, daß bei dem erfindungsgemäßen Verfahren bereits nach 5 bis 10 Minuten der Sättigungsbereich für die Bindung der Nucleinsäure erreicht ist und somit nach 5 bis 10 Minuten ein reproduzierbares Bindungsverhalten erreicht wird. Bei Verwendung eines Trägers entsprechend dem Stand der Technik (organisches aminosubstituiertes Polymer) wird dies erst nach ein bis zwei Stunden erreicht.

Die Werte der obigen Tabelle sind in Abbildung 1 dargestellt. Für die einzelnen Meßreihen wurden dazu aus der jeweiligen Radioaktivität im Überstand und dem t₀ -Wert der prozentuale Anteil der am Träger gebundenen Radioaktivität bestimmt.

## Patentansprüche

1. Verfahren zur Isolierung und Aufreinigung von Nucleinsäuren aus flüssigen Proben, **gekennzeichnet durch** folgende Verfahrensschritte:
a) Bereitstellen einer flüssigen Probe, die Nucleinsäuren enthält;
b) Bereitstellen eines Trägermaterials aus einem anorganischen hydroxylgruppenhaltigen oxidischen Material, das zumindest kristalline oder amorphe Modifikationen von SiO₂ und Silikaten, Zeolithe oder Hydroxylapatite enthält;
c) Verdünnen der Probe aus Schritt a) mit einem sauren Bindungspuffer, der keine lonen, ionische Detergenzien oder chaotrope Substanzen in Konzentrationen größer 200 mM enthält, so dass der pH-Wert der Probe auf unter pH 6 abgesenkt wird;
d) Behandeln der mittels eines Bindungspuffers angesäuerten Probe aus Schritt c) mit dem Trägermaterial aus Schritt b), wobei die Nucleinsäuren gebunden werden;
e) Abtrennen der Probe und des Bindungspuffers nach Bindung der Nucleinsäuren;
f) Eluieren der in Schritt d) gebundenen Nucleinsäuren mittels einer alkalischen Lösung.

2. Verfahren nach Anspruch 1, wobei nach Schritt e) ein Waschschritt e1) mittels eines Waschpuffers bei einem pH-Wert ≤ 6,5 ausgeführt wird.

3. Verfahren nach Anspruche 1 oder 2, **dadurch gekennzeichnet, daß** der Bindungspuffer für den Schritt c) einen pH-Wert von 3 bis 6 aufweist.

4. Verfahren nach den Ansprüchen 1 bis 3, **dadurch gekennzeichnet, daß** ein Elutionspuffer im pH-Bereich von 7,5 bis 9 verwendet wird.

5. Reagenzzusammenstellung für ein Verfahren nach den Ansprüchen 1 bis 4 zumindest enthaltend
- Trägermaterial aus einem anorganischen hydroxylgruppenhaltigen oxidischen Material, das zumindest kristalline oder amorphe Modifikationen von SiO₂ und Silikaten, Zeolithe oder Hydroxylapatite enthält, und
- einen sauren Bindungspuffer, der keine lonen, ionische Detergenzien oder chaotrope Substanzen in Konzentrationen größer 200 mM enthält.

## Claims

1. Method for the isolation and purification of nucleic acids from liquid samples, **characterised by** the following method steps:
a) provision of a liquid sample containing nucleic acids;
b) provision of a support material comprising an inorganic hydroxyl group-containing oxidic material which comprises at least crystalline or amorphous modifications of SiO₂ and silicates, zeolites or hydroxylapatites;
c) dilution of the sample from step a) with an acidic binding buffer comprising no ions, ionic detergents or chaotropic substances in concentrations greater than 200 mM, so that the pH of the sample is reduced to below pH 6;
d) treatment of the sample from step c) acidified by means of a binding buffer with the support material from step b), during which the nucleic acids are bound;
e) separation of the sample and binding buffer after binding of the nucleic acids;
f) elution of the nucleic acids bound in step d) by means of an alkaline solution.

2. Method according to Claim 1, in which, after step e), a washing step e1) is carried out by means of a washing buffer at a pH ≤ 6.5.

3. Method according to Claims 1 or 2, **characterised in that** the binding buffer for step c) has a pH of 3 to 6.

4. Method according to Claims 1 to 3, **characterised in that** an elution buffer in the pH range from 7.5 to 9 is used.

5. Reagent composition for a method according to Claims 1 to 4 at least comprising
- support material comprising an inorganic hydroxyl group-containing oxidic material which comprises at least crystalline or amorphous modifications of SiO₂ and silicates, zeolites or hydroxylapatites, and
- an acidic binding buffer which comprises no ions, ionic detergents or chaotropic substances in concentrations greater than 200 mM.

## Revendications

1. Procédé pour l'isolation et la purification d'acides nucléiques à partir d'échantillons de liquide, **caractérisé par** les étapes de procédé qui suivent:
a) fourniture d'un échantillon de liquide contenant des acides nucléiques;
b) fourniture d'un matériau de support comprenant un matériau par oxydation contenant un groupe hydroxyle inorganique, qui comprend au moins des modifications cristallines ou amorphes de SiO₂ et des silicates, des zéolites ou des hydroxylapatites;
c) dilution de l'échantillon en provenance de l'étape a) avec un tampon de liaison acide ne comprenant pas d'ions, de détergents ioniques ou de substances chaotropiques selon des concentrations supérieures à 200 mM, de telle sorte que le pH de l'échantillon soit réduit en dessous de pH 6;
d) traitement de l'échantillon en provenance de l'étape c) qui est acidifié au moyen d'un tampon de liaison avec le matériau de support en provenance de l'étape b), temps pendant lequel les acides nucléiques sont liés;
e) séparation de l'échantillon et du tampon de liaison après la liaison des acides nucléiques;
f) élution des acides nucléiques liés au cours de l'étape d) au moyen d'une solution alcaline.

2. Procédé selon la revendication 1, dans lequel, après l'étape e), une étape de lavage e1) est mise en oeuvre au moyen d'un tampon de lavage à un pH ≤ 6,5.

3. Procédé selon les revendications 1 ou 2, **caractérisé en ce que** le tampon de liaison pour l'étape c) présente un pH de 3 à 6.

4. Procédé selon les revendications 1 à 3, **caractérisé en ce qu'**un tampon d'élution selon un pH dans la plage de 7,5 à 9 est utilisé.

5. Composition de réactif pour un procédé selon les revendications 1 à 4 comprenant au moins
- un matériau de support comprenant un matériau par oxydation contenant un groupe hydroxyle inorganique, qui comprend au moins des modifications cristallines ou amorphes de SiO₂ et des silicates, des zéolites ou des hydroxylapatites, et
- un tampon de liaison acide ne comprenant pas d'ions, de détergents ioniques ou de substances chaotropiques selon des concentrations supérieures à 200 mM.
